(19) **Europäisches Patentamt**
**European Patent Office**
**Office européen des brevets**

(11) Publication number: **0 422 233 B1**

(12) # EUROPEAN PATENT SPECIFICATION

(45) Date of publication of patent specification: **11.01.95**

(51) Int. Cl.$^6$: **A61B 17/36**

(21) Application number: **90903224.5**

(22) Date of filing: **14.02.90**

(86) International application number:
**PCT/JP90/00178**

(87) International publication number:
**WO 90/09603 (23.08.90 90/20)**

(54) **LASER BEAM EMITTER.**

(30) Priority: **15.02.89 JP 35841/89**

(43) Date of publication of application:
**17.04.91 Bulletin 91/16**

(45) Publication of the grant of the patent:
**11.01.95 Bulletin 95/02**

(84) Designated Contracting States:
**AT CH DE ES FR GB IT LI NL SE**

(56) References cited:
**EP-A- 0 152 766      EP-A- 0 292 622**
**DE-A- 2 646 029      JP-A-60 149 940**
**JP-U-62 201 961      US-A- 4 209 017**
**US-A- 4 273 127**

(73) Proprietor: **S.L.T. JAPAN CO, LTD.**
**3rd Floor Tateno Bldg., 15-5**
**Iidabashi 2-chome, Chiyoda-ku**
**Tokyo 102 (JP)**

(72) Inventor: **DAIKUZONO, Norio 381, Kofudai**
**4-chome**
**Ichihara-shi**
**Chiba-ken 290-02 (JP)**

(74) Representative: **Bloch, Gérard et al**
**2, square de l'Avenue du Bois**
**F-75116 Paris (FR)**

## Description

### Technical Field

This invention relates to a laser light emitter to permit an incision, vaporization of living tissues of an animal such as a human body, a thermal therapy and the like, in case of surgical and internal medical treatments.

### Prior Art

In these days, medical treatments such as incisions of living tissues of animal organisms by irradiation with laser light are conspicuous due to its ability of hemostasis.

It had been the conventional method that the laser light was irradiated from the fore end of an optical fiber which is brought out of contact with the living tissues. But this method causes severe damage to the fore end of the optical fiber. Therefore, a method which has been utilized lately is as follows;

First, laser light is transmitted into an optical fiber, whose fore end portion locates adjacent to treated living tissues. Next, the laser light fed out from the optical fiber is fed into an emitting probe, which is brought into or out of contact with the living tissues. Then, the laser light is emitted from the surface of the probe to be irradiated against the living tissues. In this case, the probe should be brought into contact with the living tissues (hereafter "living tissue" is sometimes expressed by "tissue" only).

On the other hand, USP No. 4273127 discloses a method of an incision for treated tissues with a laser light emitter comprising a knife-shaped light guide having a working knife-edge and an optical fiber fixed to the back face of the light guide. The treated tissues are incised by the laser light, which is emitted from the fore end of the optical fiber, which goes through the light guide and which is emitted from the knife-edge. In this case, the treated tissues can be incised with laser light irradiation together with mechanical operation of the knife-edge.

However, according to this method, a distance between the fore end of the optical fiber and the knife-edge causes serious power loss. Therefore, a laser light generator having a high output power level is required.

Additionally, since an emitting direction of the laser light from the fore end of the optical fiber should reach the knife-edge of the light guide precisely, a high accuracy is required in this prior art. Further, since the emitter composes two members; optical fiber and light guide, it is very expensive to produce the emitter.

In the light guide, the laser light is scattered to be emitted. Therefore, the rate of the laser light emission from the knife-edge is reduced. It is apparently from also this fact that the laser light generator should have a high output power level for sufficient incisions.

The power distribution of the laser light is concentrated on the knife-edge which corresponds to a center line of the direction of the optical fiber's fore end. And the rate of the laser light emitted from around the knife-edge is reduced. Accordingly, the ability of an incision is influenced by a slight change of an angle made by contact of the light guide with the incised tissues. As a result, a surgery with this emitter of the prior art can not be carried out smoothly.

It is therefore a main object of the present invention to provide a laser light emitter, which permits laser light irradiation with very small power loss and laser light emission from the broad area of its external surface and which can be fabricated with low cost due to its simple composition; a laser light emitting portion is optical fiber itself.

### Disclosure of the Invention

According to the present invention, a laser light emitter comprises an optical fiber extending in a longitudinal direction and a holder supporting the optical fiber. At the fore end portion of the optical fiber, a core is not surrounded by a clad but exposed to be a laser light emitting portion. Then, the emitting portion is bending at least partially at a certain angle on a longitudinal cross section of the extending direction of the optical fiber.

In order to fix surely the optical fiber to the holder, the optical fiber has a circular cross section and extends along the inner surface of a concave groove having an arc shaped cross section formed at the edge of the substantially plate-shaped holder so as to be fixed thereto by a heat resistant adhesive.

In order to use this emitter surgically, the holder is connected to the fore end of a hand piece held by an operator. Then, the holder substantially supports the exposed core portion of the optical fiber and the hand piece supports the base portion of the optical fiber.

In order to incise tissues efficiently, the exposed core portion and at least the core-side of the holder are formed to be gradually thinner to its longitudinal tip end.

In order to select suitable means from incision with laser light irradiation and mechanical incision with a knife, the holder is plate-shaped and is provided with a knife-edge at the opposite side to its core-side.

A surface layer, which contains laser light absorbing particles, laser light scattering particles having a larger refractive index than that of the material of the core and a binder made from a laser light transmissible material, can be formed on a suitable part of the exposed surface of the optical fiber's bending portion so that the laser light can be emitted from the surface layer; a desired emitting portion.

A rough surface can be formed on at least the exposed surface of the optical fiber's bending portion and the surface layer can be formed on the rough surface so that the laser light can be emitted more efficiently with scattering.

In the emitter of the present invention, the optical fiber extends in the longitudinal direction, and at its fore end portion, its core is not surrounded by the clad but exposed to be the emitting portion. The holder supports the optical fiber. Then, at least the part of the emitting portion is bending at least partially at the certain angle on the longitudinal cross section of the extending direction of the optical fiber. In case of a straight optical fiber, the laser light is emitted from the tip end of the optical fiber. However, according to the present invention, since the core is bending, the laser light is emitted from the bending portion of the optical fiber.

Moreover, if the above mentioned surface layer is formed on the exposed surface of the optical fiber's bending portion, most of the laser light is emitted not from the tip end of the core portion, but from the surface layer, because the laser light is scattered in the surface layer. Therefore, if the emitter, which is provided with the surface layer properly, is prepared in advance, the laser light can be emitted from a suitable part having a desired size in accordance with a purpose of a medical treatment.

Further, since the laser light transmitted in the optical fiber is emitted from the optical fiber itself, laser light power is used very efficiently. As a result, the laser light generator is not required to have a high output power level.

## Brief Description of the Drawings

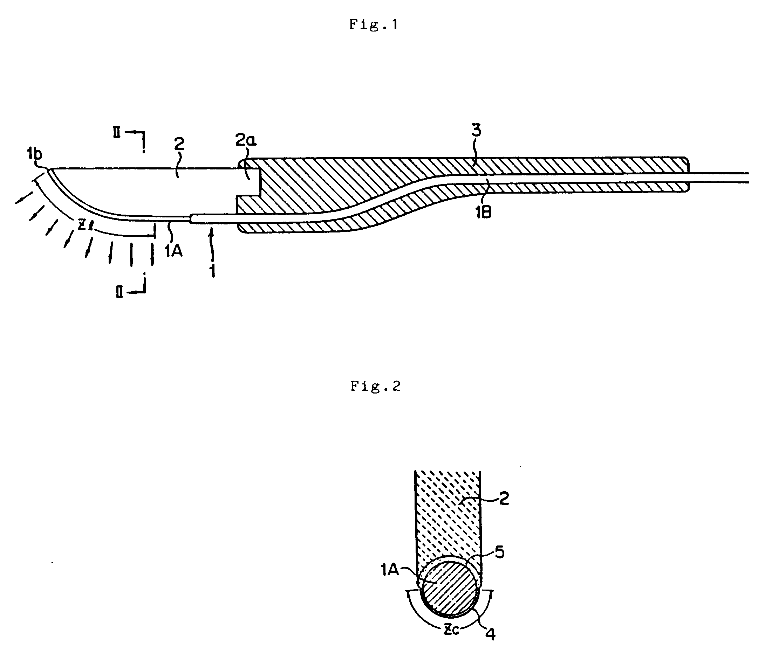

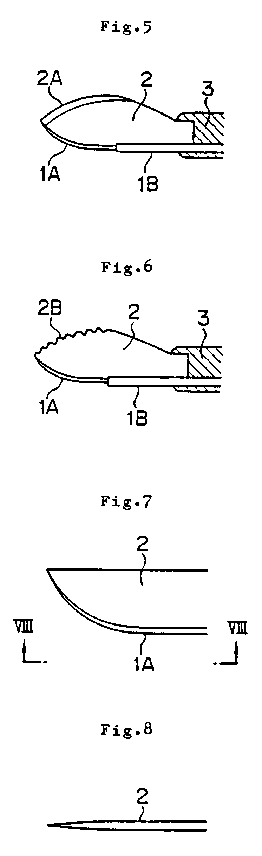

Fig. 1 is an elevational view of the first embodiment of a laser light emitter relating to the present invention; Fig. 2 is a cross sectional view taken on line II - II of Fig. 1; Figs. 3 and 4 are schematic enlarged sectional views showing embodiments of surface layers and laser light emission; Figs. 5, 6 and 7 are elevational views of other embodiments of laser light emitters; Fig. 8 is a cross sectional view taken on line VIII - VIII of Fig. 7; Figs. 9 and 10 are elevational views of still other embodiments of laser light emitters; Fig. 11 is a cross sectional view of still another embodiment showing an exposed core portion of a laser light emitter and forming of a laser light reflection layer.

## The Best Mode to Carry Out the Invention

Now, the present invention is described more particularly.

Fig. 1 shows a representative embodiment of a laser light emitter relating to the present invention. The emitter comprises an optical fiber 1, a holder 2 which supports the optical fiber 1 and a hand piece 3 which is connected to the holder 2 and which is held by an operator.

The holder 2 is fixed to the hand piece 3 by, for example, pressing of a connecting portion 2a of the holder 2 into the hand piece 3 and using an adhesive as supplement between mating surfaces. As is generally known, the optical fiber 1 comprises a core and a clad. However at the fore end portion of the optical fiber 1, the core is not surrounded by the clad but exposed to be an exposed core portion 1A. At the base portion of the optical fiber 1, the clad remains to surround the core. Further, the surface of the clad can be surrounded by a protecting tube (not shown) if desired.

The core portion 1A extends with 2/3 length of a core-side edge of the holder 2 from a tip end thereof. Then a rest of the optical fiber 1; the base of the optical fiber 1 is surrounded by the clad 1B. The core portion 1A is fixed to the edge of the holder 2. As shown in Fig. 2, the edge of the holder 2 is cut away longitudinally to have a concave groove having an arc shaped cross section. The core portion 1A is fixed along to the inner surface of the concave groove. Then heat resistant adhesive 5 is applied to the mating surfaces of the holder 2 and the core portion 1A.

The base portion of the optical fiber 1 extends along the inner side or the edge of the hand piece 3 and projects out. Then, this projecting part of the optical fiber 1 is connected to a laser light generator optically.

EP 0 422 233 B1

A controller using for adjusting the laser light power is preferably provided on the hand piece 3 or between the hand piece 3 and the laser light generator.

The fore end portion of the core portion 1A is bending at a certain angle on a longitudinal cross section of the extending direction of the core portion 1A. In Fig. 2, this longitudinal cross section is a plane of this paper. In the embodiment of Fig. 1, the core portion 1A is bending from the horizontal direction to an upper left on a plane of this paper. A surface layer 4 is formed at Zl area in the extending direction as shown in Fig. 1 and at Zc area of a circumference of the core portion on the cross section as shown in Fig. 2.

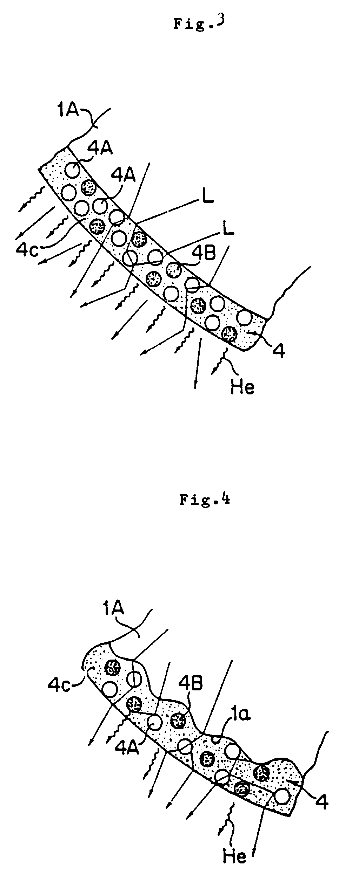

As shown in Fig. 3, the core portion 1A is covered with the surface layer 4 which contains light scattering particles 4A made of sapphire and the like having a larger refractive index than that of the core portion 1A. While the laser light L emitted from the surface of the core portion 1A passes through the surface layer 4, the laser light L, which impinges on the light scattering particle 4A, is partially reflected on the surface of the light scattering particle 4A or is partially penetrates into and is emitted from the particle 4A with refraction. Therefore, the laser light L is emitted in various directions from the entire surface layer 4. This produces a broad area of laser light irradiation.

Further, the surface layer 4 contains laser light absorbing particles 4B made of carbon and the like. Accordingly, when the laser light L impinges on the laser light absorbing particle 4B, the greater part of the energy of the laser light L is converted to heat energy He by means of the laser light absorbing particle 4B, and the tissues are heated by the heat energy from the surface layer 4.

Since the vaporization of the tissues is accelerated, the tissues can be incised with a low power level of the laser light from the core portion 1A. Therefore, when the tissues are incised, the core portion, in other words, the laser light emitter relating to the present invention can be moved rapidly. Further, the required power level of the laser light penetrating into the core portion 1A is low. As a result, the surgery can be carried in short time, further with the cheap and small scaled laser light generator.

On the other hand, referring to forming of the surface layer 4, for example, if a dispersion containing said laser light absorbing particles 4B and light scattering particles 4A is coated on the surface of the core portion 1A, after a vaporization of a dispersion medium, the contact of the emitter such as the edge of this emitter with the tissues or other substances causes damage to the surface layer 4 very easily. Because the above mentioned both kinds of particles are attached to the surface of the core portion 1A only by physical adsorptive power. Therefore, by a binder which sticks the laser light absorbing particles 4B and the light scattering particles 4A to the surface of the core portion 1A, an adhesion of the surface layer 4 to the core portion 1A is enhanced.

In this case, the binder is preferably made of light transmissible material 4C such as quartz and the like to ensure laser light penetrating in the surface layer 4. On the other hand, laser light transmissible particles having a melting point same as or lower than that of the core portion 1A are used as the transmissible material 4C and they are dispersed together with the absorbing particles 4B and the light scattering particles 4A in a proper liquid such as water. Then the core portion 1A painted with this dispersion is baked at a temperature which is higher than a melting point of the transmissible particle 4C and within a limit as the core portion 1A can keep its shape. Accordingly, the transmissible particles 4C melt to form the surface layer of high mechanical strength together with the laser light absorbing particles 4B and the light scattering particles 4A. Therefore, the damage to the surface layer can be reduced because of its high strength.

In this case, as shown in Fig. 4, a rough surface 1a is formed on the surface of the core portion 1A to give a large effect of laser light scattering.

The core portion of the optical fiber relating to the present invention is usually fabricated from quartz. Further, a natural or an artificial ceramic material such as diamond, sapphire and the like as well as halogenide glass can be used as a material of the core portion. The diameter of the core portion is preferably 10 - 1000 $\mu$m.

The light scattering particles, having the larger refractive index for the laser light than that of the core portion are of a natural or artificial material such as diamond, sapphire, quartz (a melting point is preferably high), single crystal zirconium oxide ($ZrO_2$), high melting point glass, transmissible and heat resistant synthetic resin, laser light reflective metal, and a particle which is laser light reflective or non-reflective metal particle coated with laser light reflective metal such as gold, aluminum and the like by means of the surface treatment such as plating.

The transmissible material is preferably made from the transmissible particle which can make a film when it melts and more preferably which has heat resistance such as natural or artificial, sapphire, quartz, glass, transmissible and heat resistant synthetic resin and the like. A suitable transmissible material is selected from these materials in consideration of the relation to the material of the core portion 1A.

The laser light absorbing particle is made of carbon, graphite, iron oxide, manganese dioxide and the any other materials which can absorb the laser light to generate heat energy.

4

A content of each particle in the surface layer (wt%) and each average particle size is preferably within ranges as shown in a following table. More preferable content and particle size are put in parentheses.

| | Content(wt%) | Average Particle Size($\mu$m) |
|---|---|---|
| Light Scattering Particle(A) | 90 - 1 (70 - 20) | 0.2-300 (1 - 50) |
| Absorbing Particle(B) | 90 - 1 (70 - 10) | 0.2 - 500 (1 - 100) |
| Transmissible Particle(C) | 10 - 90 (20 - 50) | 0.2 - 500 |

The thickness of the surface layer is preferably 10$\mu$m - 5mm, more preferably 30$\mu$m - 1mm. The surface layer is formed as explained hereinafter. If the surface layer having a desired thickness can not be formed by one cycle of the method, the method should be repeated until the surface having the desired thickness can be obtained.

The above mentioned three kinds of particles are dispersed in a dispersion medium, then it is heated to a temperature which is higher than the melting point of the transmissible particle, then the core portion is dipped in the heated dispersion.

Alternatively, the three kinds of particles are melted to be sprayed to the core portion.

Further, other suitable methods for forming the surface layer can be used.

By the above described first method, the dispersion dispersing the three kinds of particles can be painted to the core portion. Moreover, this painting method facilitates the operation, because what should be done in this method is that only a part, which is desired to be covered with the surface layer, of the core portion is dipped in the dispersion and pulled up therefrom. Therefore this method is practical and rational.

As the dispersion medium, suitable liquid such as water, alcohol or mixture of them can be used. Further sugar or starch is added to increase the viscosity of the dispersion medium.

As described before, according to the present invention, the forming of the surface layer 4 on the surface of the core portion extends the area of the laser light irradiation on the tissues. Because the laser light is emitted widely in many directions from the surface layer 4 as shown in Fig. 1

On the other hand, the inventor found following things; when the above mentioned percentage of (B) is high, the incision can be started with a low power level of laser light, then it is possible for the emitter to be moved quickly. However, hemostasis of the treated tissues is reduced. Therefore, the core portion with high percentage of (B) in the surface layer is used effectively for the incision of the tissues which bear damage to some extent such as skin, fat layer and the like.

On the other hand, the core portion with low percentage of (B) is useful to incise the tissues for which the hemostasis is regarded to be important such as liver, heart and the like. In this case, the power level of the output from the laser light generator must be raised and the emitter must be moved slowly.

Referring to some kinds of experiments relating to this invention, the inventor introduced these two equations, (1) and (2).

$$\frac{(B)}{(A)+(B)+(C)} \propto \frac{\text{Quantity of laser light for heating}}{\text{Incident laser energy}} \qquad (1)$$

$$\frac{(A)+(C)}{(A)+(B)+(C)} \propto \frac{\text{Quantity of laser light for transmitting}}{\text{Incident laser energy}} \qquad (2)$$

Equation (1) means that heat generation is progressed as (B) is increased, then the incision is performed by mainly vaporization. Therefore, the laser light can not penetrate into so deeply the tissues, because most of the incident laser energy is spent for heating. As a result, since the tissues are not incised deeply, the depth of the coagulation layer is reduced.

Equation (2) means that a lot of incident laser energy penetrates deeply into the tissues, then the tissues absorbing laser light is heated, therefore, the coagulation is made in the tissues.

If laser light emitters which have some kinds of core portions having different percentages of (B) in the surface layers are prepared in advance, a suitable emitter can be selected in accordance with a medical purpose. thereby a desired treatment can be carried out.

As shown in Fig. 11, in order to give concentrated laser light emission only from the external surface of the core portion 1A, a reflecting layer 6 made by plating of gold, aluminium and the like can be formed on

EP 0 422 233 B1

the back (holder side) surface of the core portion 1A and/or the core-side edge of the holder 2. Fig. 11 shows the reflecting layer 6 formed on the back surface of the core portion 1A. Fig. 11 is a cross sectional view taken on line II - II of Fig. 1 as another embodiment of Fig. 2.

A knife portion 2A of Fig. 5 and a cutting portion 2B of Fig. 6 for stripping the tissues in the form of sheets may be fabricated respectively by means of mechanical treatment. The portion 2A and 2B are formed on an edge, which is opposite to the core-side edge of the holder 2. By using such emitters, mechanical incision and stripping in the form of sheets of the tissues can be carried out as well as the incision by the laser light during a medical operation.

In Figs. 7 and 8, still another embodiment relating to the present invention is shown. According to this embodiment, the core portion 1A is tapered and the holder 2 is gradually thinner to the tip end of the holder 2. Since the core portion 1A is tapered, it is easy to progress the emitter so that the core portion can be pushed into the tissues, thereby mechanical incision can be performed. Further, even if there is no surface layer on the surface of the core portion 1A, almost all of the laser light can be emitted from the external surface including the tip end of the tapered core portion. In the embodiment of Fig. 1, if the surface layer is not formed, the rate of the laser light emission from the bending portion of the core portion would be larger than that from the straight portion of the core portion. As a result, in the present invention, the surface layer is not always necessary due to the bending portion of the core portion 1A.

It is clear that the rate of the laser light emission is increased by the formation of the surface layer in general cases. On the other hand, as shown in Fig. 1, the core portion 1A is not tapered but keeps same thickness to the tip end, which is in contrast with the tapered core portion in Figs. 7 and 8. In this case, even if the surface layer is formed on the entire surface of the core portion 1A, there is a small amount of the leak of laser light from the tip end 1b of the core portion. In order to compensate this leak, a reflecting film made by aluminum plating and the like can be formed on the tip end.

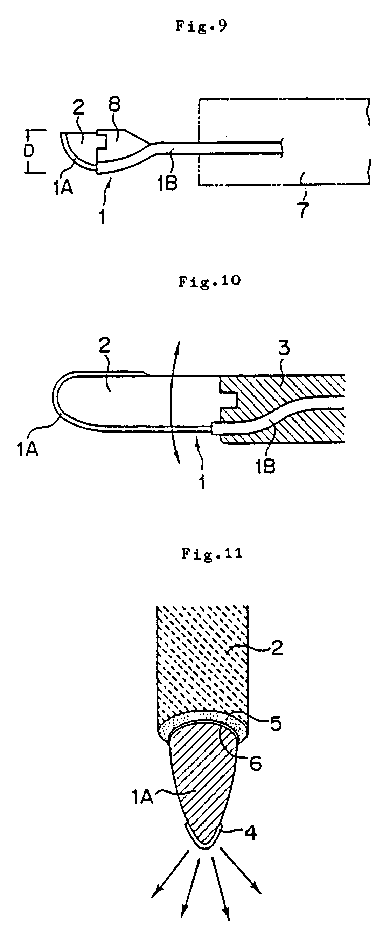

Fig. 9 shows an embodiment of the laser light emitter of the present invention which is inserted into a tube 7 of an endoscope. A height D of this emitter is preferably 0.5 - 4.0 mm. For incision with this emitter, a base portion of the optical fiber is repeated to move back and forth by an operation at outside of the tube 7. A fixing piece 8 is also provided.

In Fig. 10, the holder 2 is rounded off at its fore end, the core portion 1A extends along the edge of from one external side through the rounded fore end to a part of its other side and is fixed thereto. Using this emitter, a convex tumor can be incised by the swing of the laser light emitter in the direction indicated by the arrow in Fig. 10. In this case, forming of a surface layer is of no effect.

### Industrial Utilization

It will be appreciated from the foregoing description that, according to the present invention, the laser light emitter used for mainly the surgical treatment can be provided with advantages of laser light irradiation with very small power loss, laser light emission from the broad area and fabrication with low cost due to its simple composition; the laser light emitting portion is optical fiber itself.

### Claims

1.  A laser light emitter comprising;
    an optical fiber (1), which extends in a longitudinal direction and, at its fore end portion (1A), whose core is not surrounded by a clad but exposed to be a laser light emitting portion and is bending at least partially at a certain angle (Zl) on a longitudinal cross section of its extending direction; and
    a holder (2), which supports said optical fiber (1).

2.  A laser light emitter according to claim 1, wherein said holder (2) is made of a heat resistant material having a melting point higher than that of a material of said core.

3.  A laser light emitter according to claim 1 or 2, wherein said holder substantially is plate-shaped and has a concave groove having an arc shaped cross section at its core-side edge and said optical fiber (1) has a circular cross section and extends along the inner surface of said concave groove and is thereto by a heat resistant adhesive.

4.  A laser light emitter according to claim 1, wherein said holder is connected to the fore end of a hand piece (3) held by an operator and substantially supports said exposed core of said optical fiber (1) and said hand piece supports the base portion of said optical fiber.

6

5. A laser light emitter according to claim 1, wherein said exposed core (1A) and at least said core-side part of said holder are formed to be gradually thinner to its longitudinal tip end (1b).

6. A laser light emitter according to claim 1, 2, 3, 4 or 5, wherein said holder is plate-shaped and is provided with a knife portion (2A) at a side being opposite to said core-side.

7. A laser light emitter according to claim 1, wherein a surface layer, which contains laser light absorbing particles (4B) and laser light scattering particles (4A) having a larger refractive index than the index of the material of said core (1A), is formed on at least partially the exposed surface of the bending portion of said optical fiber (1).

8. A laser light emitter according to claim 1, wherein a surface layer which contains laser light absorbing particles (4B), laser light scattering particles (4A) having a larger refractive index than that of the material of said core and a binder made from a laser light transmissible material, is formed on at least partially the exposed surface of the bending portion (1A) of said optical fiber (1).

9. A laser light emitting probe according to claim 7 or 8, wherein a rough surface is formed on at least partially exposed surface of said bending portion (1A) of said optical fiber (1) and said surface layer is formed on said rough surface.

10. A laser light emitter according to claim 1, wherein, in said laser light emitting portion (1A) of said optical fiber (1), a laser light reflecting layer is formed on at least one surface of the holder-side surface of said optical fiber and the core-side surface of said holder.

11. A laser light emitter according to claim 1, wherein said holder (2) is substantially plate-shaped and rounded off at its fore end portion and said core extends along to be fixed to the edge from one side through the rounded fore end portion to a part of its other side.

**Patentansprüche**

1. Vorrichtung sum Aussenden von Laserlicht, umfassend : eine Lichtleitfaser (1), die sich in Längsrichtung erstreckt und deren Kern an ihrem vorderen Endteil (1A) nicht ummantelt ist, sondern freiliegt, um einen Laserlicht emittierenden Abschnitt zu bilden, und sich im Längsschnitt in Richtung seiner Längenausdehnung zumindest teilweise über einem bestimmten Winkel (Zl) biegt; und einen Halter (2), welcher die Lichtleitfaser (1) trägt.

2. Vorrichtung zum Aussenden von Laserlicht nach Anspruch 1, bei welcher del Halter (2) aus einem hitzebeständigen Material gefertigt ist, das einen höheren Schmelzpunkt besitzt als das Material des Kerns.

3. Vorrichtung zum Aussenden von Laserlicht nach Anspruch 1 oder 2, bei welcher der Haller im wesentlichen ure eine Platte geformt ist und an seiner kernseitigen Kante eine konkave Furche aufweist, die einen bogenförmigen Querschnitt besitzt, und die Lichtleitfaser (1) einen kreisförmigen Querschnitt besitzt und sich entlang der Innenfläche der konkaven Furche erstreckt und an dieser durch einen kitzebeständigen Kleber befestigt ist.

4. Vorrichtung zum Aussenden von Laserlicht nach Anspruch 1, bei welcher der Haller an dem vorderen Ende eines von einem Operateur gehaltenen Griffteils (3) angebracht ist und im wesentlichen den freiliegenden Kern der Lichtleitfaser (1) trägt und das Griffteil den Basisabschnitt der Lichtleitfaser trägt.

5. Vorrichtung zum Aussenden von Laserlicht nach Anspruch 1, bei welcher der freiliegende Kern (1A) und zumindest der kernseitige Teil des Halters so geformt sind, daß sie in Längsrichtung auf seine Endspitze (1b) zu allmählich dünner werden.

6. Vorrichtung zum Aussenden von Laserlicht nacht Anspruch 1, 2, 3, 4 oder 5, bei welcher der Haller wie eine Platte degormt ist und auf der der Kernseite entgegengesetzten Seite mit einen Klingenteil (ZA) versehen ist.

**7.** Vorrichtung zum Aussenden von Laserlicht nach Anspruch 1, bei welcher eine Oberflächenschicht, die Laserlicht absorbierende Partikel (4B) und Laserlicht streuende Partikel (4A) mit einem größeren Brechungsindex als das Material des Kerns (1A) enthält, zumindest teilweise auf der freiliegenden Oberfläche des gebogenen Abschnitts der Lichtleitfaser (1) ausgebildet ist.

**8.** Vorrichtung zum Aussenden von Laserlicht nach Anspruch 1, bei welcher eine Oberflächenschicht, die Laserlicht absorbierende Partikel (4B), Laserlicht streuende Partikel (4A) mit einem größeren Brechungsindex als das Material des Kerns und ein Bindemittel aus einem laserlichtdurchlässigen Material enthält, zumindest teilweise auf der freiliegenden Oberfläche des gebogenen Abschnitts (1A) der Lichtleitfaser (1) ausgebildet ist.

**9.** Laserstrahlsonde nach Anspruch 7 oder 8, bei welcher eine rauhe Oberfläche zumindest teilweise auf der freiliegenden Oberfläche des gebogenen Abschnitts (1A) der Lichtleitfaser (1) ausgebildet ist und die Oberflächenschicht auf dieser rauhen Oberfläche gebildet ist.

**10.** Vorrichtung zum Aussenden von Laserlicht nach Anspruch 1, bei welcher in dem Laserlicht emittierenden Abschnitt der Lichtleitfaser (1A) eine Laserlicht reflektierende Schicht auf zumindest einer Oberfläche der halterseitigen Oberfläche der Lichtleitfaser und der kernseitigen Oberfläche des Halters ausgebildet ist.

**11.** Vorrichtung zum Aussenden von Laserlicht nach Anspruche 1, bei welcher der Halter (2) im wesentlichen wie eine Platte geformt und an seinem vorderen Endteil abgerundet ist und sich der Kern von der einen Seite über den abgerundeten vorderen Endteil bis zu einem Teil auf dessen anderer Seite erstreckt und an der Konte befestigt ist.

**Revendications**

**1.** Emetteur de lumière laser comprenant
une fibre optique (1) s'étendant en direction longitudinale et, à sa partie d'extrémité avant (1A), dont le noyau n'est pas entouré par un revêtement mais exposé pour constituer une portion d'émission de lumière laser et est incurvé au moins partiellement sur un certain angle (Zℓ) de sa section longitudinale selon sa direction d'extension; et
un support (2) qui supporte ladite fibre optique (1).

**2.** Emetteur de lumière laser selon la revendication 1, dans lequel ledit support (2) est réalisé en un matériau thermorésistant ayant un point de fusion supérieur à celui du matériau dudit noyau.

**3.** Emetteur de lumière laser selon la revendication 1 ou 2, dans lequel ledit support a sensiblement la forme d'une plaque et présente une gorge concave ayant une section transversale en forme d'arc au niveau de son bord côté noyau et ladite fibre optique (1) présente une section transversale circulaire et s'étend le long de la surface intérieure de ladite gorge concave en lui étant appliquée par un adhésif thermorésistant.

**4.** Emetteur de lumière laser selon la revendication 1, dans lequel ledit support est relié à l'extrémité avant d'une pièce de manoeuvre (3) maintenue par un opérateur et supporte essentiellement ledit noyau exposé de ladite fibre optique (1) et ladite pièce à main supporte la partie de base de ladite fibre optique.

**5.** Emetteur de lumière laser selon la revendication 1, dans lequel ledit noyau exposé (1A) et au moins ladite partie côté noyau dudit support sont formés pour s'amincir progressivement vers sa pointe d'extrémité longitudinale (1b).

**6.** Emetteur de lumière laser selon la revendication 1, 2, 3, 4 ou 5, dans lequel ledit support est en forme de plaque et est pourvu d'une partie de coupe (2A) sur un côté opposé audit côté noyau.

**7.** Emetteur de lumière laser selon la revendication 1, dans lequel une couche superficielle, contenant des particules (4B) absorbant la lumière laser et des particules (4A) diffusant la lumière laser, ayant un indice de réfraction supérieur à l'indice de la matière dudit noyau (1A), est formée au moins partiellement sur la surface exposée de la partie incurvée de ladite fibre optique (1).

8. Emetteur de lumière laser selon la revendication 1, dans lequel une couche superficielle contenant des particules absorbant la lumière laser (4B), des particules diffusant la lumière laser (4A), ayant un indice de réfraction supérieur à celui de la matière dudit noyau et un liant, constitué d'une matière transmettant la lumière laser, est formée au moins partiellement sur la surface exposée de la partie incurvée (1A) de ladite fibre optique (1).

9. Sonde émettrice de lumière laser selon la revendication 7 ou 8, dans laquelle une surface rugueuse est formée au moins partiellement sur la surface exposée de ladite partie incurvée (1A) de ladite fibre optique (1) et ladite couche superficielle est formée sur ladite surface rugueuse.

10. Emetteur de lumière laser selon la revendication 1, dans lequel ladite partie émettrice de lumière laser de ladite fibre optique (1A), une couche de réflexion de lumière laser est formée sur au moins une surface de la surface côté support de ladite fibre optique et la surface côté noyau dudit support.

11. Emetteur de lumière laser selon la revendication 1, dans lequel ledit support (2) est sensiblement en forme de plaque et est bombé à sa partie d'extrémité avant et ledit noyau s'étend en étant fixé au bord depuis un côté, au-delà de la partie d'extrémité avant bombée jusqu'à une partie de l'autre côté.

Fig.1

Fig.2

EP 0 422 233 B1

Fig.3

Fig.4

Fig.5

Fig.6

Fig.7

Fig.8

Fig.9

Fig.10

Fig.11